# EUROPEAN PATENT SPECIFICATION

(11) **EP 1 288 258 B1**
(45) Date of publication and mention of the grant of the patent: **23.12.2009**
(21) Application number: 02705062.4
(22) Date of filing: 01.03.2002
(51) Int. Cl.: C08L 33/02, C08L 33/04, C08L 33/24, C08L 43/04, C08L 29/10, C08L 39/04, C08F 230/08

(54) **AQUEOUS POLYMER EMULSIONS AND COSMETICS WITH THE USE OF THE SAME**
WÄSSRIGE POLYMEREMULSIONEN UND KOSMETIKA DIE DIESE VERWENDEN
EMULSIONS POLYMERIQUES AQUEUSES ET PRODUITS COSMETIQUES UTILISANT CES EMULSIONS

(30) Priority: 02.03.2001 JP 2001058443; 06.07.2001 JP 2001206606; 06.07.2001 JP 2001206607
(43) Date of publication of application: 05.03.2003
(73) Proprietor: Shiseido Co., Ltd., Tokyo 104-8010 (JP)
(72) Inventor: KANEDA, Isamu, Shiseido Res. Ctr., (Shin-yokohama), Yokohama-shi, Kanagawa 224-8558 (JP); NAKAMURA, Ayano, Shiseido Res. Ctr., (Shin-yokoh.), Yokohma-shi, Kanagawa 224-8558 (JP); SOGABE, Atsushi, Shiseido Res. Ctr., Shin-yokoh.), Yokohma-shi, Kanagawa 224-8558 (JP); YANAKI, Toshio, Shiseido Res. Ctr., (Shin-yokoh.), Yokohama-shi, Kanagawa 224-8558 (JP)
(74) Representative: Gudat, Axel
(86) International application number: PCT/JP2002/001896
(87) International publication number: WO 2002/070604

(56) References cited:
- WO-A1-98/54255
- DE-A1- 4 341 260
- DE-A1- 19 752 436
- JP-A- 3 227 312
- JP-A- 4 321 618
- JP-A- 5 132 627
- JP-A- 6 166 613
- JP-A- 6 211 630
- JP-A- 6 211 631
- JP-A- 6 271 738
- JP-A- 7 173 404
- JP-A- 9 286 805
- US-A- 2 983 719
- US-E- R E34 958

## Description

### FIELD OF THE PRESENT INVENTION

The present invention relates to a water-based polymer emulsion and a cosmetic formulation utilizing the same, especially to the improvement of such a polymer.

### BACKGROUND OF THE PRESENT INVENTION

An organic solvent-soluble cellulose-based polymer or a vinylic polymer has mainly been employed as a film-forming agent in a cosmetic formulation. Such a film-forming agent is incorporated in a makeup cosmetic formulation such as a nail enamel, or hair styling formulation. However, a recent consciousness of the protection from the effect of the emission of such an organic solvent on a human health or from the environmental pollution, thus, encourages the development of a water-based material utilizing no organic solvents.

Under such circumstances, a utilization of a water-based polymer emulsion as a material for a cosmetic formulation has extensively been attempted, but no mechanical properties of a resulting coating film which are comparable with those of an organic solvent-soluble resin has actually been accomplished and various methods for improvement thereof have been proposed. Thus, a method for improving the mechanical properties of a coating film and for improving the film-forming property by means of microminiaturizing the particle size of the water-based polymer emulsion or imparting the water-based polymer emulsion with a core-shell morphology was proposed. Nevertheless, a known water-based polymer emulsion gives a coating film having an insufficient water resistance which leads problematically to an insufficiently durability of cosmetics in application since it gives a film which is formed just by the concentration of the polymer as a result of the drying of the formulation. Accordingly, a method was proposed in which a copolymer comprising an alkoxysilane residue-containing monomer is dispersed finely in water (Japanese Patent No.3145219). This method involves a technology in which an alcohol in an alcoholic solution is removed, then re-dispersed in water, or dispersed forcibly in the presence of a surfactant, whereby obtaining a dispersion whose mean particle size is 0.005 µm.

However, such a finely dispersed formulation requires a large amount of a surfactant and an extremely high mechanical force, which makes it difficult to accomplish an efficient production. In addition, a polymer containing an alkoxysilane residue undergoes a crosslinking during the process for distilling an alcoholic solvent off, resulting in a dispersion having a poor film-forming property and a coating film whose water resistance cannot be expected.

### SUMMARY OF THE PRESENT INVENTION

The first objective of the present invention is to provide a water-based polymer emulsion having a satisfactory storage stability and giving a coating film whose water resistance and adhesiveness are excellent. The second objective of the present invention is to provide the water-based polymer emulsion described above which has furthermore excellent characteristics for being used in a hair cosmetics.

We focused on the introduction of an ambient temperature crosslinking system and finally discovered that by copolymerizing an appropriate amount of a silane coupling monomer by emulsion polymerization an ambient temperature-crosslinking water-based polymer emulsion having a satisfactory storage stability and exhibiting excellent mechanical properties of the coating film which can not be achieved by a conventional water-based polymer emulsion can be obtained. Furthermore, it was also discovered that by using a cationic emulsifier in the water-based polymer emulsion described above a cationic water-based polymer emulsion exhibiting excellent mechanical properties when applied to a hair care formulation can be obtained, whereby establishing the present invention.

Thus, for the purpose of accomplishing the first objective described above, a water-based polymer emulsion according to the present invention is a water-based polymer emulsion obtained by emulsion polymerization of one or more silane coupling monomer selected from the group consisting of the monomers (A) represented by Formula (1), one or more lipophilic radical polymerizable monomer selected from the monomers (B) represented by Formula (2), and one or more hydrophilic radical polymerizable monomer selected from the monomers (C) represented by Formula (3), in which the polymer having the monomer (A)-derived reactive silyl group remaining therein is dispersed at a concentration or lower allowing the crosslinking between said silyl groups to be formed in a water-based dispersion medium. 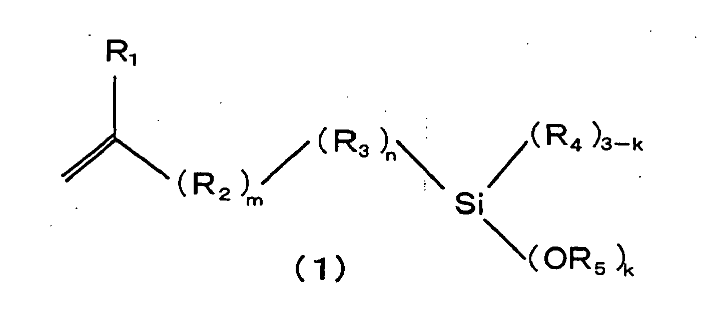
( wherein R1 is a hydrogen atom or an alkyl group having 1 to 6 carbon atoms, R2 is a group selected from 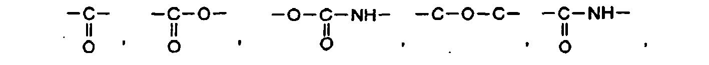
R3 is an alkylene group having 1 to 6 carbon atoms, each of R4
and R5 is a hydrogen atom or an alkyl group having 1 to 6 carbon atoms, each of m and n is 0 or 1 and k is an integer of 1 to 3.) 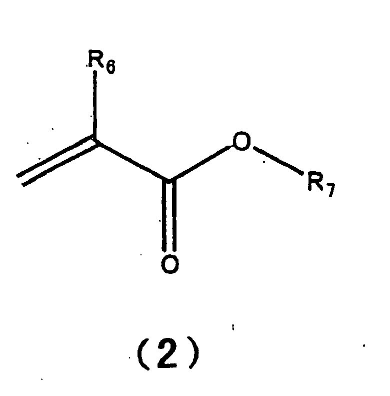
( wherein R6 is a hydrogen atom or a methyl group, R7 is an alkyl group having 1 to 18 carbon atoms, an alkyl group having a fluorine atom and 1 to 18 carbon atoms or an alkyl group having 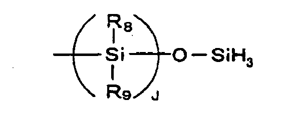
and 1 to 18 carbon atoms, in which each of R8 and R9 is a hydrogen atom or a methyl group, and J is an integer of 1 to 18.) 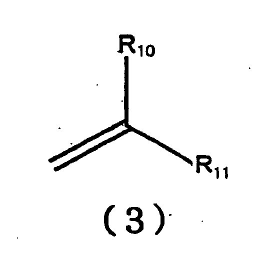
(wherein R10 is a hydrogen atom or a methyl group, R11 is a carboxyl group, ester group, ether group, amide group, urethane group and hydroxyl group and 1 to 6 carbon atoms.)

In a water-based polymer emulsion described above, the total amount of the monomers (A) is 0.001 to 10 % by mass based on the total amount of the monomers.

In a water-based polymer emulsion described above, the total amount of the monomers is preferably 20 to 60 % by mass.

In a water-based polymer emulsion described above, the total amount of the emulsifiers is preferably 0.1 to 20 % by mass based on the total amount of the monomers.

In a water-based polymer emulsion described above, the water-based polymer emulsion is preferably one resulting from emulsion polymerization by one or more reactive polymerizing agents.

A cosmetic formulation according to the present invention is a cosmetic formulation containing a water-based polymer emulsion described above.

A cosmetic formulation described above is preferably a hair treatment formulation, hair styling formulation, water-based nail polish formulation, eyeliner, mascara or sunscreen formulation.

Also for the purpose of accomplishing the second objective of the present invention, a water-based polymer emulsion according to the present invention is a water-based polymer emulsion described above which is a cationic water-based polymer emulsion obtained by emulsion polymerization using a cationic emulsifier.

A cosmetic formulation according to the present invention is a cosmetic formulation containing a cationic water-based polymer emulsion described above.

A cosmetic formulation described above is preferably a hair styling formulation, hair treatment formulation or hair rinse formulation.

### BRIEF DESCRIPTION OF THE DRAWINGS

Figure 1 is a diagram showing the dehydration condensation upon film forming of a monomer (A) according to the present invention.
Figure 2 is a diagram showing the ambient temperature crosslinking of a water-based polymer emulsion according to the present invention.
Figure 3 is a graph showing the transmittance of a sunscreen formulation containing a water-based polymer emulsion according to the present invention, as being measured before and after washing.
Figure 4 is a graph showing the relationship between the concentration of a water-based polymer emulsion according to the present invention and the increase rate of the bending stiffness of hair.
Figure 5 is a graph showing the increase rate of the bending stiffness of hair when using a cationic water-based polymer emulsion according to the present invention and an anionic water-based polymer emulsion.

### DETAILED DESCRIPTION OF THE PREFERRED EMBODIMENT

Preferred embodiments of the present invention are discussed in the following.

In the present invention, the monomers (A) to (C) form a polymer represented for example by Formula (I):

··· -A-C-A-C-B-A-C-··· (I)

as a result of emulsion polymerization. Such a polymer can exist stably in a water-based dispersion medium. Each polymer employed here, when used in the dispersion medium at a certain concentration or higher, undergoes the crosslinking as indicated for example by Formula (II): 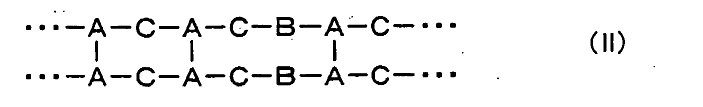 and forms a relatively firm coating film. Accordingly, when a water-based polymer emulsion of the present invention is incorporated in a cosmetic formulation at a concentration lower than the crosslinking starting level and then is allowed to be concentrated as a result of the evaporation of the dispersion medium upon application of the formulation onto a skin, each polymer is allowed to undergo the crosslinking, whereby forming a qualitatively stable and firm cosmetic coating film successfully.

A monomer (A) in the present invention is one capable of crosslinking after emulsion polymerization. In a monomer (A) represented by Formula (1) of the present invention, R₁ is a hydrogen atom or an alkyl group having 1 to 6 carbon atoms. R₂ is an ester, ether, amide urethane and an alkylene group having 1 to 6 carbon atoms. R₃ is an alkylene group having 1 to 6 carbon atoms, preferably a propylene group. R₄ is a hydrogen atom or an alkyl group having 1 to 6 carbon atoms, preferably a methyl group. OR₅ is a reactive functional group which forms a silanol group upon hydrolysis whereby effecting the crosslinking between the copolymeric molecules of the present invention, and denotes an alkoxyl group having 1 to 6 carbon atoms, preferably a methoxy group, ethoxy group or propoxy group in view of the stability of the copolymer, the safety of a by-product formed upon hydrolysis and the reactivity of the crosslinking reaction.

A monomer (A) described above is preferably a polymerizable monomer having a vinylic group, an acryloxy or methacryloxy group and an alkoxysilyl group, such as vinyltrimethoxysilane, vinyltriethoxysilane, vinyltripropoxysilane, vinylmethyldimethoxysilane, vinylmethyldiethoxysilane, vinylmethyldipropoxysilane, γ-acryloxypropyltrimethoxysilane, γ-acryloxypropyltriethoxysilane, γ-acryloxypropyltripropoxysilane, γ-acryloxypropylmethyldimethoxysilane, γ-acryloxypropylmethyldiethoxysilane, γ-acryloxypropylmethyldipropoxysilane, γ-methacryloxypropyltrimethoxysilane, γ-methacryloxypropyltrimethoxysilane, γ-methacryloxypropyltripropoxysilane, γ-methacryloxypropylmethyldimethoxysilane, γ-methacryloxypropylmethyldiethoxysilane and γ-methacryloxypropylmethyldipropoxysilane and the like. In a copolymer of the present invention, one or more of the monomers (A) described above can be employed as constituent monomers.

A monomer (A) is contained in an amount of 0.001 to 10 % by mass based on the total amount of the monomers, more preferably 0.01 to 5 % by mass. An amount less than 0.001 % by mass leads to substantially no crosslinking reaction when a cosmetic formulation is applied to a skin, hair or nail or only slight crosslinking reaction, if any, resulting in almost no improvement in the coating film strength. An amount exceeding 10 % by mass allows the crosslinking to occur continuously in a cosmetic formulation, resulting in an extremely poor stability of the polymer emulsion. Also when using as a coating film in a cosmetic formulation, the coating film should be removed at any desired time safely and conveniently. From this point of view, the amount of monomers (A) is preferably 10 % or less.

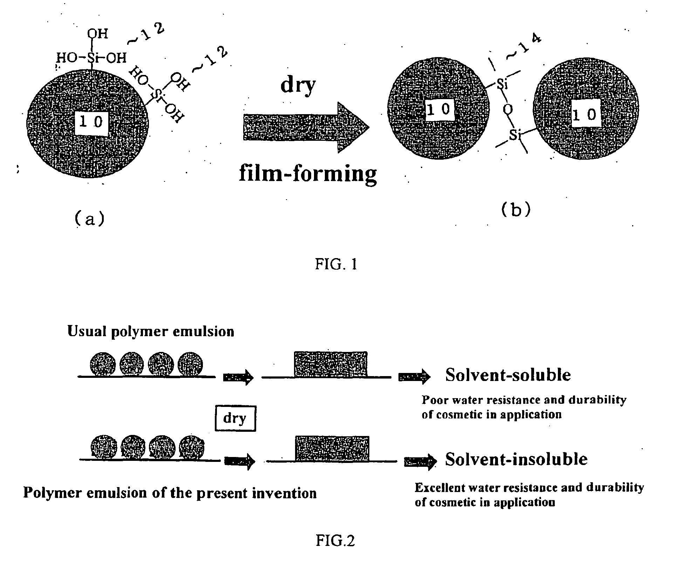

A monomer (A) forms a polymer 10 together with monomers (B) and (C) in the process of emulsion polymerization while their alkoxyl group may be hydrolyzed to form a silanol group 12 (Figure 1 (a)). While the silanol group 12 present on the surface of a polymer particle or inside of the particle rarely undergoes dehydration condensation in the state of a water dispersion in which water is present in a large excess, the silanol group 12 reacts with each other or with a functional group in the polymer capable of reacting with the silanol group 12 to undergo the dehydration condensation in a dry state upon film forming whereby establishing a crosslinking structure 14 (Figure 1 (b)).

Accordingly, as it is shown in Fig.2, a usual polymer emulsion leads to a coating film having an insufficient strength since it undergoes no crosslinking even when the dispersion medium is evaporated, and its usability as a cosmetic formulation is deteriorated extremely when the polymer concentration or polymerization degree is increased in an attempt to obtain a sufficient coating film strength. On the contrary, the present invention allows a firm coating film to be formed under the conditions involving the polymer concentration and polymerization degree which allow a satisfactory usability as a cosmetic formulation to be obtained since the polymer further undergoes the crosslinking with the evaporation of the dispersion medium.

In the present invention, a monomer (B) is for example, methyl acrylate, ethyl acrylate, n-propyl acrylate, isopropyl acrylate, n-butyl acrylate, tert-butyl acrylate, isobutyl acrylate, hexyl acrylate, octyl acrylate, 2-ethylhexyl acrylate, lauryl acrylate, cyclohexyl acrylate, isobomyl acrylate and the like. An ester of methacrylic acid may for example be methyl methacrylate, ethyl methacrylate, n-propyl methacrylate, isopropyl methacrylate, n-butyl methacrylate, tert-butyl methacrylate, isobutyl methacrylate, hexyl methacrylate, octyl methacrylate, 2-ethylhexyl methacrylate, cyclohexyl methacrylate, isobornyl methacrylate and the like.

It is also possible for the purpose of improving the usability as a cosmetic material to copolymerize a polydimethylsiloxane methacrylate, polydimethylsiloxane acrylate and a fluorine-based monomer. Styrene may also be copolymerized. In a copolymer of the present invention, one or more of the monomers (B) described above can be used as constituent monomers.

In a film forming process of a water-based polymer emulsion, the fusion between the emulsion particles is a very important factor. The fusion is improved extremely by incorporating a hydrophilic monomer (C). Typically, a non-ionic water-soluble monomer is preferred. Thus, those employed preferably as monomers (C) described above are methacrylic acid, methylvinyl ether, 2-methoxyethylvinyl ether, methyltriethylene glycol vinyl ether, 2-hydroxyethyl (meth)acrylate, pentaerythritol (meth)acrylate, 2,3-dihydroxypropyl methacrylate, hydroxyethoxyethyl methacrylate, acrylamide, N-methylacrylamide, N-ethylacrylamide, N-propylacrylamide, N,N-dimethylacrylamide, N,N-diethylacrylamide, N-acryloylpyrolidone, N-acryloylmorpholine, vinylpyrolidone, 2-acrylamide-2-methyl-1-propanesulfonic acid and the like. Also in a copolymer of the present invention, one or more of the monomers (C) described above can be used as constituent monomers.

An excessively high copolymerization ratio of a hydrophilic monomer (C) may affect adversely the water resistance of a coating film. Accordingly, the copolymerization ratio of the monomer (C) is preferably 0.01 to 30% by mass. More preferably, the ratio is 0.1 to 10 % by mass.

The combination of the monomers described above can be selected so that an excellent texture can be obtained and the glass transition temperature can desirably be adjusted. The glass transition temperature of a polymer emulsion can be calculated, for example based on the equation of FOX, from the mass ratio of the monomers to be incorporated, and can be selected within the range from -20°C to 120°C. Especially by product of the present invention, a tough film can be obtained because of the crosslinking at ambient temperature after film forming, even if the glass transition temperature of the polymer itself is low (Figure 2).

A monomer described above is added preferably in an amount of 20 to 60% by mass based on the total amount of the water-based polymer emulsion. More preferably, the amount to be added is 30 to 50% by mass. An amount less than 20% by mass leads to a constitutional disadvantage when incorporating as a cosmetic material, and also to a disadvantage in view of the shipping efficiency. An amount exceeding 60% by mass leads to an extremely increased viscosity of the polymer emulsion, which is not suitable in an actual use.

As an emulsifier employed in the present invention, a reactive emulsifier is employed preferably. By effecting emulsion polymerization using a reactive emulsifier, the emulsifier can be copolymerized with a polymer, whereby avoiding the disadvantage of a water-based polymer emulsion which is experienced as a deterioration of the coating film properties due to the emulsifier remaining in the coating film.

While a reactive emulsifier is not limited particularly, it may be those listed below which are commercially available. ADEKA REASOAP SE series and ADEKA REASOAP NE series (ASAHI DENKA CO., LTD.), AQUALON RN series and AQUALON HS series (DAI-ICHI KOGYO SEIYAKU, CO., LTD), ELEMINOL JS-2 (SANYO CHEMICAL INDUSTRIES) may be exemplified. Among these reactive emulsifiers, an allylic substance is preferred.

Furthermore, when using a water-based polymer emulsion according to the present invention in a hair cosmetic, a cationic emulsion is employed preferably as an emulsifier. Since the surface condition of hair is anionic, an efficient adhesion of the polymer particle onto the hair is expected when the polymer emulsion is converted to be cationic. This can be accomplished by effecting emulsion polymerization using a cationic emulsifier.

A cationic emulsifier is not limited particularly, and those listed below can be incorporated. For example, dodecyltrimethylammonium salts, cetyltrimethylammonium salts, decyltrimethylammonium salts, stearyltrimethylammonium salts, cetylpyridium salts, decylpyridium salts, stearylpyridinium salts, oxyalkylenetrialkylammonium salts and dioxyalkylenedialkylammonium salts and the like can be mentioned. A reactive emulsifier containing a polymerizable group in its molecule can also be incorporated. For example, allyltrialkylammonium salts and diallyldialkylammonium salts can be mentioned.

Any of the emulsifiers described above can be employed alone or in combination with each other. While the amount of an emulsifier to be incorporated may vary depending on the emulsifying ability of the emulsifier, it is preferably 0.1 to 20% by mass based on the total amount of the monomers, more preferably 1 to 10% by mass. An amount less than 0.1% by mass leads to a difficulty in ensuring the emulsion stability. An amount exceeding 20% by mass leads to a marked deterioration of the mechanical properties of the coating film.

An initiator for emulsion polymerization is preferably a water-soluble peroxide generating a radical as a result of thermal decomposition or an azo compound. For example, potassium persulfate, ammonium persulfate, sodium perchlorate, ammonium perchlorate, hydrogen peroxide and azobisaminodipropanoic acid can be exemplified. It can be used as a redox initiator by being combined with a reducing agent.

The polymerization temperature is higher than the polymerization initiation temperature of each initiator. For example, when a peroxide-based initiator is employed, a temperature of about 70°C is employed usually. While the polymerization time period is not limited particularly, it is usually 2 to 24 hours. For obtaining a polymer having a relatively high molecular weight, the reaction is continued preferably for about one day. A too short reaction time allows unreacted monomers to remain, and leads to a relatively small molecular weight. Nevertheless, care must be taken to avoid any reduction in the reaction solution volume during the reaction which may lead to the completion of the crosslinking of the silanol group derived from a monomer (A).

The molecular weight of a copolymer according to the present invention is not limited particularly, and any polymerization degree higher than that of an oligomer can allow the intended effect to be exerted. Nevertheless, the mean molecular weight is preferably 2000 to 200000 since a too low polymerization degree leads to a reduction in the coating film strength while a too high polymerization degree leads to a poor film-removing performance.

During emulsion polymerization, a suitable amount of a buffer or chain transfer agent for adjusting the polymerization degree can be incorporated in addition to various starting materials described above. When formulating a cosmetic formulation, any component which is incorporated usually into a cosmetic formulation can be incorporated if necessary as long as the effect of the present invention is not affected adversely. For example, thickner, film-forming auxiliary agent, plasticizers, humectants, UV protective agent, antiseptic and colorants can be mentioned.

A thickner is for example, an alkaline-swelling thickner such as ACULYN 22^{™} (Rohm & Haas), an association type polyurethane such as UH750^{™} (ASAHI DENKA) and ADEKA NOL GT-700^{™} (ASAHI DENKA), a carboxyvinylpolymer such as CARBOPOL 940^{™} (Goodrich), CARBOPOL 941^{™} (Goodrich), CARBOPOL 1342^{™} (Goodrich), CARBOPOL 1382^{™} (Goodrich), HIVIS WAKO^{™} (WAKO PURE CHEMICAL) and the like, a thickening polysaccharide such as xanthan gum, guar gum and hydrophobic cellulose, a clay mineral such as montmorillonite, hectorite and smectite.

A film-forming auxiliary and a plasticizer are for example, cellosolves such as cellosolve, methylcellosolve and butylcellosolve, carbitols such as carbitol, dimethylcarbitol, diethylcarbitol, butylcarbitol and dibutylcarbitol, carbonates such as ethylene carbonate and propylene carbonate, acetates such as cellosolve acetate, butylcellosolve acetate, butylcarbitol acetates and sucrose acetate, alcohols such as hexanol, benzyl alcohol and phenethyl alcohol, diols such as hexylene glycol, ethylene glycol and propylene glycol, esters such as phthalic acid diesters, adipic acid diesters, succinic acid diesters, sebacic acid diesters, abietic acid esters, capric acid esters, caproic acid esters, myristic acid esters and citric acid esters, benzoic acid esters such as sucrose benzoate as well as diethylbenzene.

The preferred examples of the present invention are described below.

### Amounts of monomers

In a water-based polymer emulsion of the present invention, the preferred amount of a monomer was verified.

### [Test Example 1 to 9]

To a 500 ml reaction vessel fitted with a stirrer, reflux condenser, thermometer and nitrogen inlet, 150 parts of an ion exchanged water, 0.1 parts of sodium carbonate and 5 parts of ELEMINOL JS-2^{™} were added and dissolved while adjusting the pH. After purging with nitrogen for 30 minutes, 0.3 parts of ammonium persulfate was dissolved in, whereby obtaining (I).

A monomer mixture solution consisting of 10 parts of styrene, 60 parts of methyl methacrylate, 30 parts of n-butyl acrylate and 0.3 parts of γ-methacryloxypropyltrimethoxysilane was prepared and designated as (II).

(I) was heated to 70°C, and treated dropwise with (II) at 0.8 g/min over a period of about 2 hours. Subsequently, the temperature was kept at 70°C to effect maturing further for 2 hours. After maturing followed by cooling to room temperature followed by filtration, a water-based polymer emulsion was obtained. The parts (I) and (II) were employed in the respective amounts shown in Table 1. Hereinafter all amounts are in % by mass. Based on the following evaluation criteria, the stability and the film-forming ability were judged as any of the three degrees shown below.

### [Evaluation criteria]

○: Good
△: Slightly poor
×: Poor

**Table 1**

| | Test Example | | | | | | | | |
|---|---|---|---|---|---|---|---|---|---|
| | 1 | 2 | 3 | 4 | 5 | 6 | 7 | 8 | 9 |
| (I) | 10 | 20 | 30 | 40 | 50 | 60 | 70 | 80 | 90 |
| (II) | 90 | 80 | 70 | 60 | 50 | 40 | 30 | 20 | 10 |
| Stability | × | × | × | ○ | ○ | ○ | ○ | ○ | ○ |
| Film-forming ability | - | - | - | ○ | ○ | ○ | ○ | ○ | △ |

As it is evident from Table 1, the water-based polymer emulsion whose total monomer content was 20 to 60% by mass exhibited excellent stability and film-forming ability, while one having a total monomer content of 20% by mass or less tended to be poorly film-forming and one having a total monomer content of 60% by weight or more underwent gelling gradually. Accordingly, the total monomer amount is preferably 20 to 60% by mass.

### Amount of monomer (A) based on total monomer amount

In a water-based polymer emulsion of the present invention, the preferred amount of a monomer (A) based on the total monomer amount was verified.

### [Test Example 10 to 15]

To a 500 ml reaction vessel fitted with a stirrer, reflux condenser, thermometer and nitrogen inlet, 150 parts of an ion exchanged water, 0.1 parts of sodium carbonate and 5 parts of ELEMINOL JS-2^{™} were added and dissolved while adjusting the pH. After purging with nitrogen for 30 minutes, 0.3 parts of ammonium persulfate were dissolved in, and the mixture was heated to 70°C, and treated dropwise with a monomer mixture solution consisting of 10 parts of styrene, 60 parts of methyl methacrylate, 30 parts of n-butyl acrylate and a certain amount of γ-methacryloxypropyltrimethoxysilane at 0.8 g/min over a period of about 2 hours. After adding the monomers, the temperature was kept at 70°C to effect maturing further for 2 hours. After maturing followed by cooling to room temperature followed by filtration, a water-based polymer emulsion was obtained.

### [Test Example 16]

To a 500 ml reaction vessel fitted with a stirrer, reflux condenser, thermometer and nitrogen inlet, 150 parts of an ion exchanged water, 0.1 parts of sodium carbonate and 3 parts of sodium dodecylsulfate were added and dissolved while adjusting the pH. After purging with nitrogen for 30 minutes, 0.3 parts of ammonium persulfate were dissolved in and then the mixture was heated to 70°C, and treated dropwise with a monomer mixture solution consisting of 50 parts of n-butyl methacrylate, 45 parts of 2-ethylhexyl acrylate and 5 parts of methacrylic acid at 0.8 g/min over a period of about 2 hours. After adding the monomers, the temperature was kept at 70°C to effect maturing further for 2 hours. After maturing followed by cooling to room temperature followed by filtration, a water-based polymer emulsion was obtained.

### [Test Example 17]

Except for using a monomer mixture solution consisting of 55 parts of n-butyl methacrylate and 45 parts of 2-ethylhexyl acrylate as a monomer mixture solution, a water-based polymer emulsion was obtained similarly to Test Example 16.

### [Test Example 18]

Except for using a monomer mixture solution consisting of 55 parts of n-butyl methacrylate, 45 parts of 2-ethylhexyl acrylate and 0.3 parts of γ-methacryloxypropyltrimethoxysilane as a monomer mixture solution, a water-based polymer emulsion was obtained similarly to Test Example 16.

### [Test Example 19]

To a 500 ml reaction vessel fitted with a stirrer, reflux condenser, thermometer and nitrogen inlet, 150 parts of an ion exchanged water, 0.1 parts of sodium carbonate and 5 parts of ELEMINOL JS-2^{™} were added and dissolved while adjusting the pH. After purging with nitrogen for 30 minutes, 0.3 parts of ammonium persulfate were dissolved in and then the mixture was heated to 70°C, and treated dropwise with a monomer mixture solution consisting of 65 parts of n-butyl methacrylate, 30 parts of 2-ethylhexyl acrylate, 5 parts of methacrylic acid and 0.3 parts of γ-methacryloxypropyltrimethoxysilane at 0.8 g/min over a period of about 2 hours. After adding the monomers, the temperature was kept at 70°C to effect maturing further for 2 hours. After maturing followed by cooling to room temperature followed by filtration, a water-based polymer emulsion was obtained.

### [Test Example 20]

Except for using a monomer mixture solution consisting of 60 parts of n-butyl methacrylate, 30 parts of 2-ethylhexyl acrylate, 5 parts of methacrylic acid, 5 parts of X-24-8201^{™} (silicone-based monomer, SHINETSU KAGAKU) and 0.3 parts of γ-methacryloxypropyltrimethoxysilane as a monomer mixture solution, a water-based polymer emulsion was obtained similarly to Test Example 19.

### [Test Example 21]

Except for using a monomer mixture solution consisting of 60 parts of n-butyl methacrylate, 30 parts of 2-ethylhexyl acrylate, 5 parts of methacrylic acid, 5 parts of tetrafluoropropyl methacrylate and 0.3 parts of γ-methacryloxypropyltrimethoxysilane as a monomer mixture solution, a water-based polymer emulsion was obtained similarly to Test Example 19.

### [Test Example 22]

Except for using a monomer mixture solution consisting of 60 parts of n-butyl methacrylate, 30 parts of 2-ethylhexyl acrylate, 5 parts of methacrylic acid, 5 parts of heptadecafluoro-n-decyl acrylate and 0.3 parts of γ-methacryloxypropyltrimethoxysilane as a monomer mixture solution, a water-based polymer emulsion was obtained similarly to Test Example 19.

### [Test Example 23]

Except for using a monomer mixture solution consisting of 60 parts of n-butyl methacrylate, 30 parts of 2-ethylhexyl acrylate, 5 parts of methacrylic acid, 2.5 parts of X-24-8201^{™} (silicone-based monomer, SHINETSU KAGAKU), 2.5 parts of heptatecafluoro-n-decyl acrylate and 0.3 parts of γ-methacryloxypropyltrimethoxysilane as a monomer mixture solution, a water-based polymer emulsion was obtained similarly to Test Example 19.

The characteristics of the water-based polymer emulsions obtained in Test Example 10 to 23 described above are shown in Table 2.

**Table 2**

| | Test Example | | | | | | | | | | | | | |
|---|---|---|---|---|---|---|---|---|---|---|---|---|---|---|
| | 10 | 11 | 12 | 13 | 14 | 15 | 16 | 17 | 18 | 19 | 20 | 21 | 22 | 23 |
| Amount of; | | | | | | | | | | | | | | |
| monomer (A) | 0 | 0.03 | 0.3 | 3 | 10 | 12 | 0 | 0 | 0.3 | 0.3 | 0.3 | 0.3 | 0.3 | 0.3 |
| monomer (B) | 90 | 90 | 90 | 90 | 90 | 90 | 95 | 100 | 100 | 95 | 95 | 95 | 95 | 95 |
| monomer (C) | 10 | 10 | 10 | 10 | 10 | 10 | 5 | 0 | 0 | 5 | 5 | 5 | 5 | 5 |
| Stability | ○ | ○ | ○ | ○ | ○ | × | ○ | ○ | ○ | ○ | ○ | ○ | ○ | ○ |
| (50oC 1month) | (gelation) | | | | | | | | | | | | | |

As it is evident from Table 2, each water-based polymer emulsions of Test Example 11 to 14, 18 to 23 containing 0.03 to 10% by mass of the monomer (A) based on the total monomer amount and of Test Example 10, 16 and 17 containing no monomer (A) exhibited an excellent stability of the product, while Test Example 15 containing 12% by mass of the monomer (A) exhibited a poor stability and underwent gelling. This may be due to the condensation of the monomer (A) during the polymerization which leaded to the crosslinking.

### Comparison of coating film characteristics

The coating film characteristics of the water-based polymer emulsions of Test Example 10 to 14, 16 to 24 were compared. As shown in Table 3, the formulations containing the water-based polymer emulsions of Test Example 10 to 14, 16 to 24 were prepared. A glass plate was coated with each emulsion using a 0.35 mm applicator, dried at room temperature for 24 hours, and the resultant film was examined for the water resistance and the adhesiveness.

### [Test Example 24]

To a 500 ml reaction vessel fitted with a stirrer, reflux condenser, thermometer and nitrogen inlet, 150 parts of ethanol and 0.1 parts of benzoyl peroxide were added and dissolved while adjusting the pH. The mixture was heated to 70°C, and treated dropwise with a monomer mixture solution consisting of 5 parts of methacrylic acid, 65 parts of methyl methacrylate, 30 parts of n-butyl acrylate and 0.3 parts of γ-methacryloxypropyltrimethoxysilane at 0.8 g/min over a period of about 2 hours. After adding the monomers, the temperature was kept at 70°C to effect maturing further for 2 hours. After maturing, 150 parts of the ion exchanged water was added, and ethanol was distilled off, and then the mixture was cooled to room temperature and filtered to obtain a water-based polymer emulsion.

### [Water resistance evaluation criteria]

The condition of a coating film after immersing in water for 30 minutes was evaluated visually.
○: No whitening or peeling was observed.
△: Slight whitening and peeling were observed.
×: Marked whitening and peeling were observed.

### [Adhesiveness evaluation criteria]

Using a cross-cut tester, 100 square pieces were formed by cutting into a 1-mm lattice, and then peeled using an adhesive tape, and the number of the remaining pieces were counted.
O: The number of the remaining squares was 100 to 90.
△: The number of the remaining squares was 90 to 60.
×: The number of the remaining squares was less than 60.

**Table 3**

| | Formulation Example | | | | | | | | | | | | | |
|---|---|---|---|---|---|---|---|---|---|---|---|---|---|---|
| | 1 | 2 | 3 | 4 | 5 | 6 | 7 | 8 | 9 | 10 | 11 | 12 | 13 | 14 |
| Test Ex.10 | 80 | - | - | - | - | - | - | - | - | - | - | - | - | - |
| Test Ex.11 | - | 80 | - | - | - | - | - | - | - | - | - | - | - | - |
| Test Ex.12 | - | - | 80 | - | - | - | - | - | - | - | - | - | - | - |
| Test Ex.13 | - | - | - | 80 | - | - | - | - | - | - | - | - | - | - |
| Test Ex.14 | - | - | - | - | 80 | - | - | - | - | - | - | - | - | - |
| Test Ex.16 | - | - | - | - | - | 80 | - | - | - | - | - | - | - | - |
| Test Ex.17 | - | - | - | - | - | - | 80 | - | - | - | - | - | - | - |
| Test Ex.18 | - | - | - | - | - | - | - | 80 | - | - | - | - | - | - |
| Test Ex.19 | - | - | - | - | - | - | - | - | 80 | - | - | - | - | - |
| Test Ex.20 | - | - | - | - | - | - | - | - | - | 80 | - | - | - | - |
| Test Ex.21 | - | - | - | - | - | - | - | - | - | - | 80 | - | - | - |
| Test Ex.22 | - | - | - | - | - | - | - | - | - | - | - | 80 | - | - |
| Test Ex.23 | - | - | - | - | - | - | - | - | - | - | - | - | 80 | - |
| Test Ex.24 | - | - | - | - | - | - | - | - | - | - | - | - | - | 80 |
| Cellosolb | 10 | 10 | 10 | 10 | 10 | 10 | 10 | 10 | 10 | 10 | 10 | 10 | 10 | 10 |
| Acetyltributyl | 4 | 4 | 4 | 4 | 4 | 4 | 4 | 4 | 4 | 4 | 4 | 4 | 4 | 4 |
| citrate | | | | | | | | | | | | | | |
| ACULYN22 | 0.5 | 0.5 | 0.5 | 0.5 | 0.5 | 0.5 | 0.5 | 0.5 | 0.5 | 0.5 | 0.5 | 0.5 | 0.5 | 0.5 |
| Ion exchanged water | To100 | | | | | | | | | | | | | |
| Water resistance | × | ○ | ○ | ○ | ○ | △ | × | △ | ○ | ○ | ○ | ○ | ○ | × |
| Adhesiveness | × | ○ | ○ | ○ | ○ | × | × | ○ | ○ | ○ | ○ | ○ | ○ | × |

As it is evident from Table 3, each of the formulations of Formulation Examples 2 to 5 and 9 to 13 containing the water-based polymer emulsions of Test Example 11 to 14 and 19 to 23 obtained by copolymerizing the monomers (A), (B) and (C) was excellent significantly in terms of both of the water resistance and the close contact performance when compared with the formulations of Formulation Examples 1 and 6 containing the water-based polymer emulsions of Experiments 10 and 16 obtained by copolymerizing only the monomers (B) and (C), the formulation of Formulation Example 7 containing the water-based polymer emulsion of Test Example 17 obtained by copolymerizing only the monomer (B) and the formulation of Formulation Example 8 containing the water-based polymer emulsions of Test Example 18 obtained by copolymerizing only the monomers (A) and (B).

On the other hand, the formulation of Formulation Example 14 containing the water-based polymer emulsion of Test Example 24 which was not obtained by emulsion copolymerization was poor in terms of both of the water resistance and the close contact performance, possibly because the silanol group derived from the monomer (A) entirely underwent the formation of the crosslinking during the process for distilling the alcohol solvent off.

### Nail polish formulation

Table 4 shows as the examples of cosmetic the compositions of the nail polish formulations containing the water-based polymer emulsions of Test Example 10, 12, 17 and 19 described above together with the results of the evaluation. These Examples were examined for their performance in the using test by 15 experienced panelists. The evaluation items were gloss, durability of cosmetic in application (peeling resistance) and removability, each of which was judged as any of 4 degrees.

### [Evaluation criteria]

⊚: Very good
○: Good
△: Slightly poor
×: Poor

**Table 4**

| | Formulation Example | | | | |
|---|---|---|---|---|---|
| | 15 | 16 | 17 | 18 | 19 |
| Water-based polymer emulsion | | | | | |
| Test Ex.12 | 80 | - | - | - | - |
| Test Ex.19 | - | 80 | - | - | - |
| Test Ex.23 | - | - | 80 | - | - |
| Test Ex.10 | - | - | - | 80 | - |
| Test Ex.17 | - | - | - | - | 80 |
| Cellosolb | 10 | 3 | 3 | 10 | 3 |
| Acetyltributyl | 4 | 0 | 0 | 4 | 0 |
| citrate | | | | | |
| Deep marine | 1 | 1 | 1 | 1 | 1 |
| ACULYN22 | 0.5 | 0.5 | 0.5 | 0.5 | 0.5 |
| Ion exchanged water | To100 | | | | |
| Gloss | ⊚ | ⊚ | ⊚ | × | × |
| Durability of cosmetic in application | ⊚ | ⊚ | ⊚ | × | × |
| Removability* | ⊚ | ⊚ | ⊚ | ⊚ | ⊚ |

| | | | | | |
|---|---|---|---|---|---|
| * Examination upon ordinary cleansing cream for their removal facileness | | | | | |

As it is evident from Table 4, each of the nail polish formulations of Formulation Example 15, 16 and 17 containing the water-based polymer emulsions of Test Example 12, 19 and 23 obtained by copolymerizing the monomers (A), (B) and (C) was extremely excellent in terms of both of the gloss and the durability of cosmetic and exhibited a satisfactory the removability, when compared with the formulations of Formulation Examples 18 and 19 containing the water-based polymer emulsions of Test Example 10 and 17 obtained by copolymerizing only the monomers (B) and (C).

### Eyeliner

Table 5 shows as the examples of the cosmetic the compositions of the eyeliner formulations containing the water-based polymer emulsions of Test Example 16, 17, 19 and 23 described above together with the results of the evaluation. These Examples were examined for their performance in the using test by 17 experienced panelists. The evaluation items were water resistance, durability of cosmetic in application and removability, each of which was judged as any of 4 degrees.

**Table 5**

| | Formulation Example | | | |
|---|---|---|---|---|
| | 20 | 21 | 22 | 23 |
| Water-based polymer emulsion | | | | |
| Test Ex.19 | 45 | - | - | - |
| Test Ex.23 | - | 45 | - | - |
| Test Ex.16 | - | - | 45 | - |
| Test Ex.17 | - | - | - | 45 |
| Iron oxide (black) | 14 | 14 | 14 | 14 |
| Glycerin | 5 | 5 | 5 | 5 |
| Polyoxyethylene (20) sorbitan | 1 | 1 | 1 | 1 |
| mono oleic acid ester | | | | |
| Carboxymethylcellulose | 0.15 | 0.15 | 0.15 | 0.15 |
| Acetyltributyl citrate | 1 | 1 | 1 | 1 |
| Ion exchanged water | To100 | | | |
| Water resistance | ⊚ | ⊚ | △ | × |
| Durability of cosmetic | ⊚ | ⊚ | × | × |
| in application | | | | |
| Removability* | ⊚ | ⊚ | ○ | ⊚ |

| | | | | |
|---|---|---|---|---|
| * Examination upon ordinary cleansing cream for their removal facileness | | | | |

As it is evident from Table 5, each of the eyeliner formulations of Formulation Example 20 and 21 containing the water-based polymer emulsions of Test Example 19 and 23 obtained by copolymerizing the monomers (A), (B) and (C) was extremely excellent in terms of both of the water resistance and the durability of cosmetic and exhibited a satisfactory removability, when compared with the eyeliner formulations of Formulation Examples 22 and 23 containing the water-based polymer emulsions of Test Example 16 obtained by copolymerizing only the monomer (B) and of Test Example 17 obtained by copolymerizing only the monomers (B) and (C).

### Mascara

Table 6 shows as the examples of the cosmetic the compositions of the mascara formulations containing the water-based polymer emulsions of Test Example 16, 17, 19 and 21 described above together with the results of the evaluation. These Examples were examined for their performance in the using test by 14 experienced panelists. The evaluation items were water resistance, durability of cosmetic in application and removability, each of which was judged as any of 4 degrees.

**Table 6**

| | Formulation Example | | | |
|---|---|---|---|---|
| | 24 | 25 | 26 | 27 |
| Water-based polymer emulsion | | | | |
| Test Ex.19 | 30 | - | - | - |
| Test Ex.21 | - | 30 | - | - |
| Test Ex.16 | - | - | 30 | - |
| Test Ex.17 | - | - | - | 30 |
| Iron oxide (black) | 10 | 10 | 10 | 10 |
| Trimethylsiloxysilicate | 20 | 20 | 20 | 20 |
| (Decamethylcyclopentasiloxane 50% solution) | | | | |
| Paraffin | 5 | 5 | 5 | 5 |
| Isoparaffin | 50 | 50 | 50 | 50 |
| Polyoxyethylene(20) cetyl ether | 2 | 2 | 2 | 2 |
| Carbitol | 2 | 2 | 2 | 2 |
| Ion exchanged water | To100 | | | |
| Water resistance | ⊚ | ⊚ | △ | × |
| Durability of cosmetic | ⊚ | ⊚ | × | × |
| in application | | | | |
| Removability* | ⊚ | ○ | ⊚ | ⊚ |

| | | | | |
|---|---|---|---|---|
| * Examination upon ordinary cleansing cream for their removal facileness | | | | |

As it is evident from Table 6, each of the mascara formulations of Formulation Example 24 and 25 containing the water-based polymer emulsions of Test Example 19 and 21 obtained by copolymerizing the monomers (A), (B) and (C) was extremely excellent in terms of both of the water resistance and the durability of cosmetic and exhibited a satisfactory removability, when compared with the mascara formulations of Formulation Examples 26 and 27 containing the water-based polymer emulsions of Experiment 16 obtained by copolymerizing only the monomers (B) and (C) and of Experiment 17 obtained by copolymerizing only the monomer (B).

### Sunscreen formulation

Tables 7 and 8 show as the examples of the cosmetic the compositions of the sunscreen formulations containing the water-based polymer emulsions of Test Example 16, 19 and 23 described above together with the results of the evaluation. These Examples were examined for their performance in the using test by 15 experienced panelists and also in terms of the water resistance. The water resistance test was conducted as described below.

TRANSPORE tape^{™} was coated with a sample at 2 mg/cm², applied onto the rim of a beaker, to which water was poured and stirred for 30 minutes, and then the tape was taken out of the beaker and subjected to the determination of in vitro SPF.

The *in vitro* SPF was obtained by irradiating the TRANSPORE tape^{™} with an Xe lamp, measuring the transmitting light using a spectroradiometer, integrating the transmitting light, and calculating an SPF from the integrated value. Based on the ratio between the SPF values before and after immersion in water, the water resistance was evaluated.

The water resistance of the sunscreen formulations of Formulation Test Example 29 and 30 was evaluated also by the measurement of the transmittance as described below.

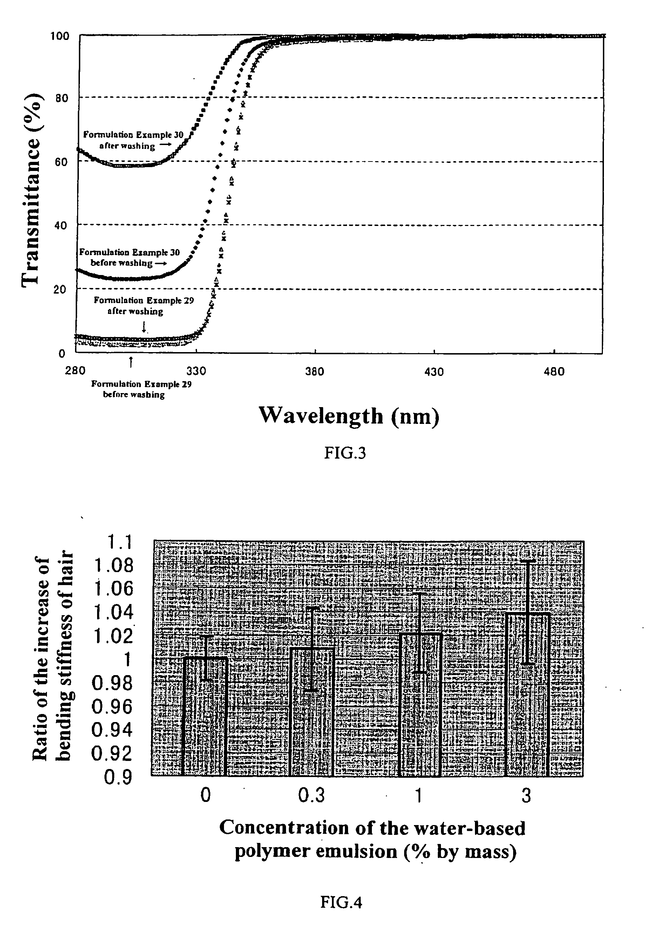

First, each sunscreen formulation was applied onto a nylon plate, and the transmittance was measured. Then the plate was washed with running water for 30 minutes, and then the transmittance was measured. The results are shown in Figure 3.

**Table 7**

| O/W type | | | |
|---|---|---|---|
| | Formulation Example | | |
| | 28 | 29 | 30 |
| Test Ex.19 | 2 | - | - |
| Test Ex.23 | - | 2 | - |
| Test Ex.16 | - | - | 2 |
| Ethanol | 10 | 10 | 10 |
| 1,3-butyleneglycol | 5 | 5 | 5 |
| Polyoxyethylene hydrogenated | 1.8 | 1.8 | 1.8 |
| castor oil | | | |
| Polydimethylsiloxane | 15 | 15 | 15 |
| Octyl methoxycinnamate | 8 | 8 | 8 |
| Isostearic acid | 2 | 2 | 2 |
| Perfume, Antiseptic | q.s. | q.s. | q.s. |
| Ion exchanged water | To100 | | |
| Spreadability | ⊚ | ⊚ | ⊚ |
| Durability of cosmetic in application | ⊚ | ⊚ | × |
| Water resistance (%) | 87 | 92 | 21 |

**Table 8**

| W/O type | | | |
|---|---|---|---|
| | Formulation Example | | |
| | 31 | 32 | 33 |
| Test Ex.19 | 2 | - | - |
| Test Ex.23 | - | 2 | - |
| Test Ex.16 | - | - | 2 |
| 1,3-butyleneglycol | 5 | 5 | 5 |
| Polyoxyethylene methylpolysiloxane | 1 | 1 | 1 |
| copolymer | | | |
| Polydimethylsiloxane | 25 | 25 | 25 |
| Octyl methoxycinnamate | 8 | 8 | 8 |
| Isostearic acid | 0.5 | 0.5 | 0.5 |
| Microparticle titanium oxide (30mµ) | 15 | 15 | 15 |
| Benton38^{™} | 0.5 | 0.5 | 0.5 |
| Perfume, Antiseptic | q.s. | q.s. | q.s. |
| Ion exchanged water | To100 | | |
| Spreadability | ⊚ | ⊚ | ⊚ |
| Durability of cosmetic | ⊚ | ⊚ | × |
| in application | | | |
| Water resistance (%) | 86 | 93 | 45 |

As it is evident from Tables 7 and 8, each of the sunscreen formulations of Formulation Example 28, 29, 31 and 32 containing the water-based polymer emulsions of Test Example 19 and 23 obtained by copolymerizing the monomers (A), (B) and (C) was extremely excellent in terms of both of the water resistance and the durability of cosmetic, when compared with the sunscreen formulations of Formulation Examples 30 and 33 containing the water-based polymer emulsions of Test Example 16 obtained by copolymerizing only the monomers (B) and (C).

Also as it is evident from Figure 3, the sunscreen formulation of Formulation Example 29 exhibited a low transmittance before washing when compared with the sunscreen formulation of Formulation Example 30, and this low transmittance of the sunscreen formulation of Formulation Example 29 was still kept even after washing unlike to the sunscreen formulation of Formulation Example 30 whose transmittance was increased after washing.

Accordingly, the sunscreen formulation containing the water-based polymer emulsion of the present invention was proven to protect the skin effectively from ultraviolet light.

### Hair spray formulation

Table 9 shows as the examples of the cosmetic the compositions of the hair spray formulations containing the water-based polymer emulsions of Test Example 16 to 23 described above together with the results of the evaluation. These Examples were examined for their performance in the 4-scale using test by 15 experienced panelists and also in terms of the water resistance and the rate of increase of the bending stiffness of hair. The water resistance was evaluated in the curl retention test using hair strand. In this method, an Example formulation was applied onto hair, which was then dried using a drier, and allowed to stand at 40°C and 75% RH for 4 hours, after which the hanging down of the hair was measured.

The rate of increase of the bending stiffness was evaluated by measuring the torsional rigidity of a single hair and calculating the rate of increase in the rigidity when comparing with a non-treated sample.

Formulation Examples and the results are as shown in Table 9.

**Table 9**

| Hair spray formulation | | | | | | | | | |
|---|---|---|---|---|---|---|---|---|---|
| | | Formulation Example | | | | | | | |
| | | 34 | 35 | 36 | 37 | 38 | 39 | 40 | 41 |
| Based solution | | | | | | | | | |
| Test Ex.19 | | 15.0 | - | - | - | - | - | - | - |
| Test Ex.20 | | - | 15.0 | - | - | - | - | - | - |
| Test Ex.21 | | - | - | 15.0 | - | - | - | - | - |
| Test Ex.22 | | - | - | - | 15.0 | - | - | - | - |
| Test Ex.23 | | - | - | - | - | 15.0 | - | - | - |
| Test Ex.16 | | - | - | - | - | - | 15.0 | - | - |
| Test Ex.17 | | - | - | - | - | - | - | 15.0 | - |
| Test Ex.18 | | - | - | - | - | - | - | - | 15.0 |
| Dipropyleneglycol | | 3.00 | 3.00 | 3.00 | 3.00 | 3.00 | 3.00 | 3.00 | 3.00 |
| Ethanol | | 15.0 | 15.0 | 15.0 | 15.0 | 15.0 | 15.0 | 15.0 | 15.0 |
| Polydimethylsiloxane | | 0.50 | 0.50 | 0.50 | 0.50 | 0.50 | 0.50 | 0.50 | 0.50 |
| Perfume, Antiseptic | | q.s. | q.s. | q.s. | q.s. | q.s. | q.s. | q.s. | q.s. |
| Ion exchanged water | | To100 | | | | | | | |
| Filling up formulation | Based solution | | | 99.5 | | | | | |
| | Compressed nitrogen | | | 0.5 | | | | | |
| Smoothness | | ○ | ⊚ | ⊚ | ⊚ | ⊚ | △ | × | △ |
| Gloss | | ⊚ | ⊚ | ⊚ | ⊚ | ⊚ | △ | × | × |
| Setting ability | | ⊚ | ⊚ | ⊚ | ⊚ | ⊚ | ○ | ○ | ⊚ |
| Bending stiffness | | ⊚ | ⊚ | ⊚ | ⊚ | ⊚ | × | × | △ |
| Water resistance (curl retention rate %) | | 95 | 93 | 94 | 95 | 92 | 25 | 26 | 54 |
| Rate of increase of Bending stiffness | | 1.055 | 1.053 | 1.056 | 1.058 | 1.055 | 1.002 | 1.000 | 1.056 |

As it is evident from Table 9, each of the hair spray formulations of Formulation Example 34 to 38 containing the water-based polymer emulsions of Test Example 18 to 22 obtained by copolymerizing the monomers (A), (B) and (C) was excellent significantly in terms of the water resistance, the rate of increase of the bending stiffness and organoleptic properties, when compared with the hair spray formulations containing the water-based polymer emulsions of Test Example 16 obtained by copolymerizing only the monomers (B) and (C), of Test Example 17 obtained by copolymerizing only the monomer (B) and of Test Example 18 obtained by copolymerizing only the monomers (A) and (B).

Furthermore, the bending stiffness effects of the inventive water-based polymer emulsions on hair were compared.

First, the torsional rigidity of a single hair before treatment was measured. Subsequently, the hair was immersed in a 0 to 3% dispersion of the polymer emulsion of Test Example 19, and then dried using a drier. The hair was then immersed in tap water for 2 hours, and dried overnight at 25°C and 50% humidity. The torsional rigidity of the single hair after treatment was measured, and the rate of increase in the rigidity when compared with the value before treatment was used to evaluate the rate of increase of the bending stiffness. The results are shown in Figure 4.

As it is evident from Figure 4, the use of the water-based polymer emulsion of the present invention resulted in a concentration-dependent increase in the bending stiffness of a hair.

The preferred working examples of the water-based polymer emulsion according to the present invention are shown in the following.

| Working Example 1 O/W sunprotector | |
|---|---|
| (formulation) | mass(%) |
| Dimethylpolysiloxane | 3.00 |
| Dioctyl succinate | 3.00 |
| Octyl methoxycinnamate | 7.00 |
| Carboxyvinylpolymer | 0.30 |
| PEMULEN TR-2^{™}(Goodrich) | 0.10 |
| Glycerin | 10.00 |
| Hydrogenated castor oil | 0.50 |
| Micro particle silica | 0.50 |
| Polymer emulsion of Test Example 19 | 2.00 |
| (as well as possible to use the polymer emulsion of Test Example 20-23) | |
| Antiseptic | q.s. |
| pH modifier | q.s. |
| Perfume | q.s. |
| Ion exchanged water | To 100 |
| | |

| Working Example 2 O/W sunscreen | |
|---|---|
| (formulation) | mass(%) |
| Dimethylpolysiloxane | 5.00 |
| Decamethylcyclopentasiloxane | 20.00 |
| Polyoxyethylene methylpolysiloxane copolymer | 4.00 |
| 1,3-butyleneglycol | 5.00 |
| Cetyl octanoate | 7.00 |
| Sorbitan sesquiisostearate | 0.50 |
| Dextrin palmitate treated micro particle zinc oxide | 10.00 |
| Micro particle titanium oxide | 5.00 |
| Dimethyldistearylammonium hectorite | 1.00 |
| Spherical polymethylmethacrylate resin | 4.00 |
| Polymer emulsion of Test Example 19 | 3.00 |
| (as well as possible to use the polymer emulsion of Test Example 20-23) | |
| Antiseptic | q.s. |
| pH modifier | q.s. |
| Perfume | q.s. |
| Ion exchanged water | To 100 |
| | |

| Working Example 3 Hair cream | |
|---|---|
| (formulation) | mass(%) |
| Dimethylpolysiloxane | 5.00 |
| Isoparaffin | 7.00 |
| Ethanol | 5.00 |
| Glycerin | 5.00 |
| Polyoxypropylene decaglyceryl ether | 2.00 |
| Isostearic acid | 1.00 |
| Hydrogenated castor oil | 0.50 |
| Imidazolium betaine | 3.00 |
| Xanthan gum | 0.50 |
| Carboxyvinylpolymer | 0.50 |
| Polymer emulsion of Test Example 23 | 3.00 |
| (as well as possible to use the polymer emulsion of Test Example 19-22) | |
| Antiseptic | q.s. |
| pH modifier | q.s. |
| Perfume | q.s. |
| Ion exchanged water | To 100 |
| | |

| Working Example 4 Hair treatment | |
|---|---|
| (formulation) | mass(%) |
| Dimethylpolysiloxane | 3.00 |
| Amino-modified silicone | 0.60 |
| Poyoxyethylene methylpolysiloxane copolymer | 0.50 |
| Ethanol | 10.00 |
| Propyleneglycol | 6.00 |
| Xanthan gum | 0.10 |
| Carboxyvinylpolymer | 0.30 |
| PEMULEN TR-2^{™}(Goodrich) | 0.10 |
| Polymer emulsion of Test Example 23 | 2.00 |
| (as well as possible to use the polymer emulsion of Test Example 19-22) | |
| Antiseptic | q.s. |
| pH modifier | q.s. |
| Perfume | q.s. |
| Ion exchanged water | To 100 |

### Cationic water-based polymer emulsions

Furthermore, in a water-based polymer emulsion of the present invention, the suitability to a hair formulation when using a cationic emulsifier during polymerization to form a cationic water-based polymer emulsion was verified.

### [Test Example 25]

To a 500 ml reaction vessel fitted with a stirrer, reflux condenser, thermometer and nitrogen inlet, 150 parts of an ion exchanged water, 0.1 parts of sodium carbonate and 3 parts of dodecyltrimethylammonium chloride were added and dissolved while adjusting the pH. After purging with nitrogen for 30 minutes, 0.3 parts of ammonium persulfate were dissolved in and then the mixture was heated to 70°C, and treated dropwise with a monomer mixture solution consisting of 65 parts of n-butyl methacrylate, 30 parts of 2-ethylhexyl acrylate, 5 parts of hydroxyethyl methacrylate and 0.3 parts of γ-methacryloxypropyltrimethoxysilane at 0.8 g/min over a period of about 2 hours. After adding the monomers, the temperature was kept at 70°C to effect maturing further for 2 hours. After maturing followed by cooling to room temperature followed by filtration, a cationic water-based polymer emulsion whose % solid mass was 39% was obtained.

### [Test Example 26]

Except for using a monomer mixture solution consisting of 60 parts of n-butyl methacrylate, 30 parts of 2-ethylhexyl acrylate, 5 parts of polydimethylsiloxane methacrylate (X-24-8201^{™}:SHINETSU KAGAKU), 5 parts of hydroxyethyl methacrylate and 0.3 parts of γ-methacryloxypropyltrimethoxysilane as a monomer mixture solution, a cationic water-based polymer emulsion whose % solid mass was 39% was obtained similarly to Test Example 25.

### [Test Example 27]

Except for using a monomer mixture solution consisting of 60 parts of n-butyl methacrylate, 30 parts of 2-ethylhexyl acrylate, 5 parts of 2,2,3,3-tetrafluoropropyl methacrylate, 5 parts of hydroxyethyl methacrylate and 0.3 parts of γ-methacryloxypropyltrimethoxysilane as a monomer mixture solution, a cationic water-based polymer emulsion whose % solid mass was 39% was obtained similarly to Test Example 25.

### [Test Example 28]

Except for using a monomer mixture solution consisting of 60 parts of n-butyl methacrylate, 30 parts of 2-ethylhexyl acrylate, 5 parts of 1H,1H,2H,2H-heptafluoro-n-decyl acrylate, 5 parts of hydroxyethyl methacrylate and 0.3 parts of γ-methacryloxypropyltrimethoxysilane as a monomer mixture solution, a cationic water-based polymer emulsion whose % solid mass was 39% was obtained similarly to Test Example 25.

### [Test Example 29]

Except for using a monomer mixture solution consisting of 60 parts of n-butyl methacrylate, 30 parts of 2-ethylhexyl acrylate, 2.5 parts of polydimethylsiloxane methacrylate (X-24-8201^{™}:SHINETSU KAGAKU), 2.5 parts of 1H,1H,2H,2H-heptafluoro-n-decyl polydimethylsiloxane acrylate, 5 parts of hydroxyethyl methacrylate and 0.3 parts of γ-methacryloxypropyltrimethoxysilane as a monomer mixture solution, a cationic water-based polymer emulsion whose % solid mass was 39% was obtained similarly to Test Example 25.

### [Test Example 30]

Except for using a monomer mixture solution consisting of 65 parts of n-butyl methacrylate and 35 parts of 2-ethylhexyl acrylate as a monomer mixture solution, a cationic water-based polymer emulsion whose % solid mass was 39% was obtained similarly to Test Example 25.

### [Test Example 31]

Except for using a monomer mixture solution consisting of 65 parts of n-butyl methacrylate, 30 parts of 2-ethylhexyl acrylate and 5 parts of hydroxyethyl methacrylate as a monomer mixture solution, a cationic water-based polymer emulsion whose % solid mass was 39% was obtained similarly to Test Example 25.

### [Test Example 32]

Except for using a monomer mixture solution consisting of 65 parts of n-butyl methacrylate, 35 parts of 2-ethylhexyl acrylate and 0.3 parts of γ-methacryloxypropyltrimethoxysilane as a monomer mixture solution, a cationic water-based polymer emulsion whose % solid mass was 39% was obtained similarly to Test Example 25.

The performance of a cosmetic formulation containing a water-based polymer emulsion was evaluated.

The water resistance was evaluated in the curl retention test using a hair strand. In this method, a formulation of Formulation Example was applied onto a hair, which was then dried using a drier, and allowed to stand at 75% humidity and 40°C for 4 hours, after which the hanging down of the hair was measured.

The rate of increase of the bending stiffness was evaluated by measuring the torsional rigidity of a single hair and calculating the rate of increase in the rigidity when comparing with a non-treated sample.

The organoleptic evaluation was made in accordance with the following evaluation criteria with judging as any of 4 degrees.

### [Evaluation criteria]

⊚: Very good
○: Good
△: Moderate
×: Poor

Formulation Examples and the results are shown in Tables 10 to 15. Hereinafter all amounts are in % by mass.

**Table10**

| Hair treatment lotion | | | | | | | | | |
|---|---|---|---|---|---|---|---|---|---|
| | | Formulation Example | | | | | | | |
| | | 42 | 43 | 44 | 45 | 46 | 47 | 48 | 49 |
| Test Ex.25 | | 9.00 | - | - | - | - | - | - | - |
| Test Ex.26 | | - | 9.00 | - | - | - | - | - | - |
| Test Ex.27 | | - | - | 9.00 | - | - | - | - | - |
| Test Ex.28 | | - | - | - | 9.00 | - | - | - | - |
| Test Ex.29 | | - | - | - | - | 9.00 | - | - | - |
| Test Ex.30 | | - | - | - | - | - | 9.00 | - | - |
| Test Ex.31 | | - | - | - | - | - | - | 9.00 | - |
| Test Ex.32 | | - | - | - | - | - | - | - | 9.00 |
| 1,3-butyleneglycol | | 3.00 | 3.00 | 3.00 | 3.00 | 3.00 | 3.00 | 3.00 | 3.00 |
| Glycerin | | 1.00 | 1.00 | 1.00 | 1.00 | 1.00 | 1.00 | 1.00 | 1.00 |
| Polyoxyethylene hydrogenated castor oil | | 0.50 | 0.50 | 0.50 | 0.50 | 0.50 | 0.50 | 0.50 | 0.50 |
| Dimethylpolysiloxane | | 1.00 | 1.00 | 1.00 | 1.00 | 1.00 | 1.00 | 1.00 | 1.00 |
| Perfume, Antiseptic | | q.s. | q.s. | q.s. | q.s. | q.s. | q.s. | q.s. | q.s. |
| Ethanol | | 10.0 | 10.0 | 10.0 | 10.0 | 10.0 | 10.0 | 10.0 | 10.0 |
| Ion exchanged water | | To100 | | | | | | | |
| Smoothness | | ○ | ⊚ | ⊚ | ⊚ | ⊚ | △ | ○ | △ |
| Gloss | | ⊚ | ⊚ | ⊚ | ⊚ | ⊚ | × | ○ | × |
| Comb usability | | ⊚ | ⊚ | ⊚ | ⊚ | ⊚ | × | △ | × |
| Bending stiffness | | ⊚ | ⊚ | ⊚ | ⊚ | ⊚ | △ | △ | ○ |
| Water resistance (curl retention rate %) | | 95 | 93 | 94 | 92 | 92 | 42 | 38 | 93 |
| Rate of increase of Bending stiffness | | 1.050 | 1.047 | 1.053 | 1.052 | 1.062 | 1.023 | 0.998 | 1.053 |

**Table11**

| Hair gel | | | | | | | | |
|---|---|---|---|---|---|---|---|---|
| | Formulation Example | | | | | | | |
| | 50 | 51 | 52 | 53 | 54 | 55 | 56 | 57 |
| Test Ex.25 | 9.00 | - | - | - | - | - | - | - |
| Test Ex.26 | - | 9.00 | - | - | - | - | - | - |
| Test Ex.27 | - | - | 9.00 | - | - | - | - | - |
| Test Ex.28 | - | - | - | 9.00 | - | - | - | - |
| Test Ex.29 | - | - | - | - | 9.00 | - | - | - |
| Test Ex.30 | - | - | - | - | - | 9.00 | - | - |
| Test Ex.31 | - | - | - | - | - | - | 9.00 | - |
| Test Ex.32 | - | - | - | - | - | - | - | 9.00 |
| ADEKA NOL GT700^{™} | 1.00 | 1.00 | 1.00 | 1.00 | 1.00 | 1.00 | 1.00 | 1.00 |
| Glycerin | 0.50 | 0.50 | 0.50 | 0.50 | 0.50 | 0.50 | 0.50 | 0.50 |
| Sodium hydroxide | q.s. | q.s. | q.s. | q.s. | q.s. | q.s. | q.s. | q.s. |
| Perfume, Antiseptic | q.s. | q.s. | q.s. | q.s. | q.s. | q.s. | q.s. | q.s. |
| Ethanol | 10.0 | 10.0 | 10.0 | 10.0 | 10.0 | 10.0 | 10.0 | 10.0 |
| Ion exchanged water | To100 | | | | | | | |
| Smoothness | ○ | ⊚ | ⊚ | ⊚ | ⊚ | △ | ○ | △ |
| Gloss | ⊚ | ⊚ | ⊚ | ⊚ | ⊚ | × | ○ | × |
| Comb usability | ⊚ | ⊚ | ⊚ | ⊚ | ⊚ | × | △ | × |
| Bending stiffness | ⊚ | ⊚ | ⊚ | ⊚ | ⊚ | △ | △ | ○ |
| Water resistance (curl retention rate %) | 94 | 93 | 95 | 93 | 95 | 39 | 35 | 91 |
| Rate of increase of Bending stiffness | 1.049 | 1.055 | 1.052 | 1.057 | 1.052 | 1.001 | 1.010 | 1.057 |

**Table12**

| Hair cream | | | | | | | | |
|---|---|---|---|---|---|---|---|---|
| | Formulation Example | | | | | | | |
| | 58 | 59 | 60 | 61 | 62 | 63 | 64 | 65 |
| Test Ex.25 | 9.00 | - | - | - | - | - | - | - |
| Test Ex.26 | - | 9.00 | - | - | - | - | - | - |
| Test Ex.27 | - | - | 9.00 | - | - | | - | - |
| Test Ex.28 | - | - | - | 9.00 | - | - | - | - |
| Test Ex.29 | - | - | - | - | 9.00 | - | - | - |
| Test Ex.30 | - | - | - | - | - | 9.00 | - | - |
| Test Ex.31 | - | - | - | - | - | - | 9.00 | - |
| Test Ex.32 | - | - | - | - | - | - | - | 9.00 |
| Liquid paraffin | 10.0 | 10.0 | 10.0 | 10.0 | 10.0 | 10.0 | 10.0 | 10.0 |
| Vaseline | 2.00 | 2.00 | 2.00 | 2.00 | 2.00 | 2.00 | 2.00 | 2.00 |
| Polyoxyethylene hydrogenated | 2.00 | 2.00 | 2.00 | 2.00 | 2.00 | 2.00 | 2.00 | 2.00 |
| castor oil | | | | | | | | |
| ADEKA NOL GT700^{™} | 0.70 | 0.70 | 0.70 | 0.70 | 0.70 | 0.70 | 0.70 | 0.70 |
| Xanthan gum | 0.10 | 0.10 | 0.10 | 0.10 | 0.10 | 0.10 | 0.10 | 0.10 |
| pH modifier | q.s. | q.s. | q.s. | q.s. | q.s. | q.s. | q.s. | q.s. |
| Perfume, Antiseptic | q.s. | q.s. | q.s. | q.s. | q.s. | q.s. | q.s. | q.s. |
| Ion exchanged water | To100 | | | | | | | |
| Smoothness | ○ | ⊚ | ⊚ | ⊚ | ⊚ | △ | ○ | △ |
| Gloss | ⊚ | ⊚ | ⊚ | ⊚ | ⊚ | × | ○ | × |
| Comb usability | ⊚ | ⊚ | ⊚ | ⊚ | ⊚ | × | △ | × |
| Bending stiffness | ⊚ | ⊚ | ⊚ | ⊚ | ⊚ | △ | △ | ○ |
| Water resistance (curl retention rate %) | 95 | 94 | 95 | 94 | 93 | 41 | 34 | 95 |
| Rate of increase of Bending stiffness | 1.055 | 1.052 | 1.056 | 1.053 | 1.055 | 1.008 | 0.999 | 1.052 |

**Table13**

| Hair mousse | | | | | | | | | |
|---|---|---|---|---|---|---|---|---|---|
| | | Formulation Example | | | | | | | |
| | | 66 | 67 | 68 | 69 | 70 | 71 | 72 | 73 |
| Based solution | | | | | | | | | |
| Test Ex.25 | | 9.00 | - | - | - | - | - | - | - |
| Test Ex.26 | | - | 9.00 | - | - | - | - | - | - |
| Test Ex.27 | | - | - | 9.00 | - | - | - | - | - |
| Test Ex.28 | | - | - | - | 9.00 | - | - | - | - |
| Test Ex.29 | | - | - | - | - | 9.00 | - | - | - |
| Test Ex.30 | | - | - | - | - | - | 9.00 | - | - |
| Test Ex.31 | | - | - | - | - | - | - | 9.00 | - |
| Test Ex.32 | | - | - | - | - | - | - | - | 9.00 |
| Dipropyleneglycol | | 5.00 | 5.00 | 5.00 | 5.00 | 5.00 | 5.00 | 5.00 | 5.00 |
| Ethanol | | 10.0 | 10.0 | 10.0 | 10.0 | 10.0 | 10.0 | 10.0 | 10.0 |
| Polydimethylsiloxane | | 5.00 | 5.00 | 5.00 | 5.00 | 5.00 | 5.00 | 5.00 | 5.00 |
| Polyoxyethylene hydrogenated | | 1.00 | 1.00 | 1.00 | 1.00 | 1.00 | 1.00 | 1.00 | 1.00 |
| castor oil | | | | | | | | | |
| Perfume, Antiseptic | | q.s. | q.s. | q.s. | q.s. | q.s. | q.s. | q.s. | q.s. |
| Ion exchanged water | | To100 | | | | | | | |
| Filling up formulation | Based solution | | | 90 | | | | | |
| | liquefied gas | | | 10 | | | | | |
| Smoothness | | ○ | ⊚ | ⊚ | ⊚ | ⊚ | △ | ○ | △ |
| Gloss | | ⊚ | ⊚ | ⊚ | ⊚ | ⊚ | × | ○ | × |
| Comb usability | | ⊚ | ⊚ | ⊚ | ⊚ | ⊚ | × | △ | × |
| Bending stiffness | | ⊚ | ⊚ | ⊚ | ⊚ | ⊚ | △ | △ | ○ |
| Water resistance (curl retention rate %) | | 94 | 93 | 96 | 93 | 95 | 44 | 42 | 93 |
| Rate of increase of Bending stiffness | | 1.053 | 1.056 | 1.054 | 1.049 | 1.051 | 0.988 | 1.005 | 1.054 |

**Table14**

| Hair spray | | | | | | | | | |
|---|---|---|---|---|---|---|---|---|---|
| | | Formulation Example | | | | | | | |
| | | 74 | 75 | 76 | 77 | 78 | 79 | 80 | 81 |
| Based solution | | | | | | | | | |
| Test Ex.25 | | 15.0 | - | - | - | - | - | - | - |
| Test Ex.26 | | - | 15.0 | - | - | - | - | - | - |
| Test Ex.27 | | - | - | 15.0 | - | - | - | - | - |
| Test Ex.28 | | - | - | - | 15.0 | - | - | - | - |
| Test Ex.29 | | - | - | - | - | 15.0 | - | - | - |
| Test Ex.30 | | - | - | - | - | - | 15.0 | - | - |
| Test Ex.31 | | - | - | - | - | - | - | 15.0 | - |
| Test Ex.32 | | - | - | - | - | - | - | - | 15.0 |
| Dipropyleneglycol | | 3.00 | 3.00 | 3.00 | 3.00 | 3.00 | 3.00 | 3.00 | 3.00 |
| Ethanol | | 15.0 | 15.0 | 15.0 | 15.0 | 15.0 | 15.0 | 15.0 | 15.0 |
| Polydimethylsiloxane | | 0.50 | 0.50 | 0.50 | 0.50 | 0.50 | 0.50 | 0.50 | 0.50 |
| Perfume, Antiseptic | | q.s. | q.s. | q.s. | q.s. | q.s. | q.s. | q.s. | q.s. |
| Ion exchanged water | | To100 | | | | | | | |
| Filling up formulation | Based solution | | | 99.5 | | | | | |
| | Compressed nitrogen | | | 0.5 | | | | | |
| Smoothness | | ○ | ⊚ | ⊚ | ⊚ | ⊚ | △ | ○ | △ |
| Gloss | | ⊚ | ⊚ | ⊚ | ⊚ | ⊚ | × | ○ | × |
| Comb usability | | ⊚ | ⊚ | ⊚ | ⊚ | ⊚ | ○ | ○ | ⊚ |
| Bending stiffness | | ⊚ | ⊚ | ⊚ | ⊚ | △ | △ | △ | ○ |
| Water resistance (curl retention | rate %) | 95 | 93 | 94 | 95 | 92 | 37 | 45 | 94 |
| Rate of increase of Bending | stiffness | 1.051 | 1.054 | 1.057 | 1.061 | 1.053 | 1.004 | 1.003 | 1.056 |

**Table 15**

| Hair rinse | | | | | | | | |
|---|---|---|---|---|---|---|---|---|
| | Formulation Example | | | | | | | |
| | 82 | 83 | 84 | 85 | 86 | 87 | 88 | 89 |
| Test Ex.25 | 7.00 | - | - | - | - | - | - | - |
| Test Ex.26 | - | 7.00 | - | - | - | - | - | - |
| Test Ex.27 | - | - | 7.00 | - | - | - | - | - |
| Test Ex.28 | - | - | - | 7.00 | - | - | - | - |
| Test Ex.29 | - | - | - | - | 7.00 | - | - | - |
| Test Ex.30 | - | - | - | - | - | 7.00 | - | - |
| Test Ex.31 | - | - | - | - | - | - | 7.00 | - |
| Test Ex.32 | - | - | - | - | - | - | - | 7.00 |
| Dimethylpolysiloxane | 2.00 | 2.00 | 2.00 | 2.00 | 2.00 | 2.00 | 2.00 | 2.00 |
| Liquid paraffin | 0.70 | 0.70 | 0.70 | 0.70 | 0.70 | 0.70 | 0.70 | 0.70 |
| Cetyl alcohol | 1.50 | 1.50 | 1.50 | 1.50 | 1.50 | 1.50 | 1.50 | 1.50 |
| Stearyl alcohol | 1.00 | 1.00 | 1.00 | 1.00 | 1.00 | 1.00 | 1.00 | 1.00 |
| Steryltrimethylanmonium chloride | 0.60 | 0.60 | 0.60 | 0.60 | 0.60 | 0.60 | 0.60 | 0.60 |
| Glycerin | 2.00 | 2.00 | 2.00 | 2.00 | 2.00 | 2.00 | 2.00 | 2.00 |
| ADEKA NOL GT700^{™} | 0.70 | 0.70 | 0.70 | 0.70 | 0.70 | 0.70 | 0.70 | 0.70 |
| Perfume, Antiseptic | q.s. | q.s. | q.s. | q.s. | q.s. | q.s. | q.s. | q.s. |
| Ion exchanged water | To100 | | | | | | | |
| Smoothness | ○ | ⊚ | ⊚ | ⊚ | ⊚ | △ | ○ | △ |
| Gloss | ⊚ | ⊚ | ⊚ | ⊚ | ⊚ | × | ○ | × |
| Comb usability | ○ | ⊚ | ⊚ | ⊚ | ⊚ | × | △ | × |
| Bending stiffness | ⊚ | ⊚ | ⊚ | ⊚ | ⊚ | △ | △ | ○ |

As it is evident from Tables 10 to 15, each of the formulations containing the cationic water-based polymer emulsions of Test Example 25 to 29 obtained by copolymerizing the monomers (A), (B) and (C) was excellent significantly in terms of the water resistance, the rate of increase of the bending stiffness and organoleptic properties, when compared with the formulations containing the cationic water-based polymer emulsions of Test Example 30 obtained by copolymerizing only the monomer (B), of Test Example 31 obtained by copolymerizing only the monomers (B) and (C) and of Test Example 32 obtained by copolymerizing only the monomers (A) and (B).

Such findings may be attributable to the characteristics of the inventive cationic polymer emulsion which is obtained by copolymerizing a suitable amount of a silane coupling monomer, which allows the emulsion to be stable in an aqueous dispersion state but allows the emulsion to undergo ambient temperature crosslinking as a result of the concentration upon drying and film-forming.

Subsequently, the effect of a water-based polymer emulsion employing a cationic emulsifier as an emulsifier on a bleached hair was compared with that employing an anionic emulsifier.

First, bleached hair was immersed in an ion exchanged water for 1 hour, and then dried at 25°C and 50% humidity overnight. The torsional rigidity of a single hair before treatment was measured.

Then, a 10% dispersion of the polymer emulsion of Test Example 25 was applied onto the hair 5 times using a spray, and the hair was dried at 25°C and 50% humidity overnight.

The rate of increase of the bending stiffness was evaluated by measuring the torsional rigidity of a single hair after treatment and calculating the rate of increase in the rigidity when comparing with the hair before treatment.

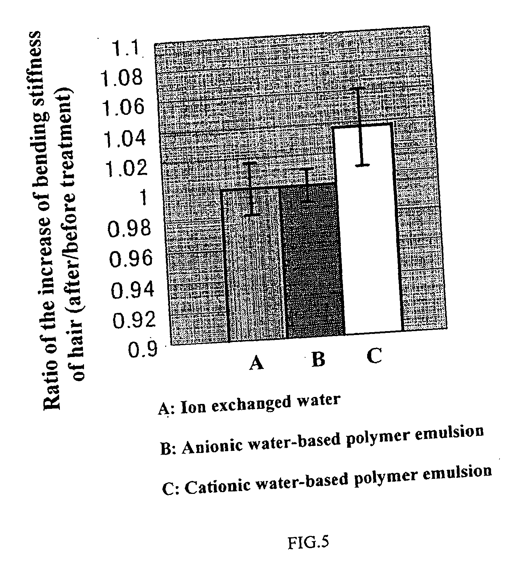

For comparison, the ion exchanged water was used to perform the test similarly. In addition, a polymer emulsion was prepared similarly to Test Example 25 except for using ELEMINOL JS-2 (anionic emulsifier) instead of cetyltrimethylammonium bromide (cationic emulsifier), and the similar test was performed using a 10% dispersion of this polymer emulsion. The results are shown in Figure 5.

As it is evident from Figure 5, the use of the cationic water-based polymer emulsion resulted in a marked increase in the effect when compared with the use of the anionic water-based polymer emulsion or water.

While an anionic water-based polymer emulsion can impart the bending stiffness to normal hair by forming a coating film on the hair as indicated in the results described above (Figure 4), it can not form a coating film on a bleached hair because of the increased anionic condition of the surface of the hair when compared with the normal condition due to cysteic acid produced on the surface of the hair. On the contrary, a cationic water-based polymer emulsion can be adsorbed more readily onto a highly anionic hair alter bleaching and can form a satisfactory coating film, resulting in a capability of imparting the bending stiffness even to such hair. Accordingly, a cationic water-based polymer emulsion exerts a more excellent effect on bleached hair when compared with an anionic water-based polymer emulsion.

As described above, it was proven that by using a cationic emulsifier a cationic water-based polymer emulsion which is excellent also as a hair formulation can be obtained.

Preferred working examples of the cationic water-based polymer emulsion according to the present invention are shown in the following.

| Working Example 5 Hair treatment lotion | |
|---|---|
| (formulation) | mass(%) |
| Polyoxyethylene methylpolysiroxane copolymer | 0.50 |
| Ethanol | 5.00 |
| Glycerin | 4.00 |
| Stearyltrimethylammonium chloride | 0.30 |
| Polymer emulsion of Test Example 28 | 2.00 |
| (as well as possible to use the polymer emulsion of Test Example 25-27) | |
| Antiseptic | q.s. |
| Perfume | q.s. |
| Ion exchanged water | To 100 |

| Working Example 6 Hair cream | |
|---|---|
| (formulation) | mass(%) |
| Dimethylpolysiloxane | 5.00 |
| Isoparaffin | 7.00 |
| Ethanol | 5.00 |
| Glycerin | 5.00 |
| Polyoxypropylene decaglyceryl ether | 2.00 |
| Isostearic acid | 1.00 |
| Hydrogenated castor oil | 0.50 |
| Imidazolium betaine | 3.00 |
| Xanthan gum | 0.50 |
| ADEKANOL GT-700^{™} | 1.00 |
| Polymer emulsion of Test Example 28 | 3.00 |
| (as well as possible to use the polymer emulsion of Test Example | 25-27) |
| Antiseptic | q.s. |
| pH modifier | q.s. |
| Perfume | q.s. |
| Ion exchanged water | To 100 |
| | |

| Working Example 7 Hair treatment | |
|---|---|
| (formulation) | mass(%) |
| Dimethylpolysiloxane | 3.00 |
| Amino-modified silicone | 0.60 |
| Polyoxyethylene methylpolysiloxane copolymer | 0.50 |
| Ethanol | 10.00 |
| Propyleneglycol | 6.00 |
| Xanthan gum | 0.20 |
| Polyoxyethylene methylpolysiloxane copolymer | 2.00 |
| Polymer emulsion of Test Example 28 | 2.00 |
| (as well as possible to use the polymer emulsion of Test Example 25-27) | |
| Antiseptic | q.s. |
| pH modifier | q.s. |
| Perfume | q.s. |
| Ion exchanged water | To 100 |

## Claims

1. A use of a water-based polymer emulsion in the manufacturing of a cosmetic formulation, wherein the water-based polymer emulsion is obtained by emulsion polymerization of one or more silane coupling monomer selected from the group consisting of the monomers (A) represented by Formula (1), one or more lipophilic radical polymerizable monomer selected from the monomers (B) represented by Formula (2), and one or more hydrophilic radical polymerizable monomer selected from the monomers (C) represented by Formula (3), wherein the total amount of the monomers (A) is 0,001 to 10 % by mass based on the total amount of the monomers, in which the polymer having the monomer (A)-derived reactive silyl group remaining therein is dispersed at a concentration or lower allowing the crosslinking between said silyl groups to be formed in a water-based dispersion medium; 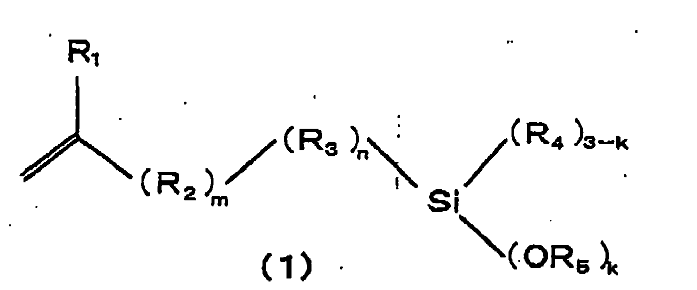
(wherein R1 is a hydrogen atom or an alkyl group having 1 to 6 carbon atoms, R2 is a group selected from 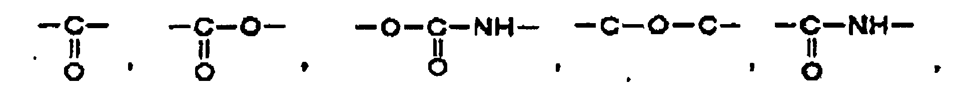
R3 is an alkylene group having 1 to 6 carbon atoms, each of R4 and R5 is a hydrogen atom or an alkyl group having 1 to 6 carbon atoms,
each of m and n is 0 or 1 and k is an integer of 1 to 3); 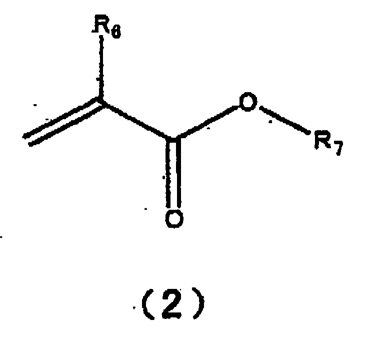
(wherein R6 is a hydrogen atom or a methyl group, R7 is an alkyl group having 1 to 18 carbon atoms, an alkyl group having a fluorine atom and 1 to 18 carbon atoms or an alkyl group having 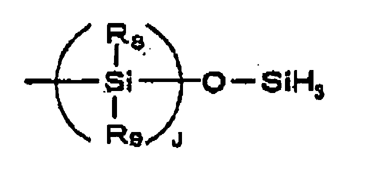
and 1 to 18 carbon atoms, in which each of R8 and R9 is a hydrogen atom or a methyl group, and J is an integer of 1 to 18); 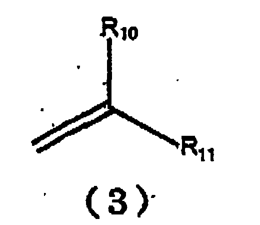
(wherein R10 is a hydrogen atom or a methyl group, R11 is a carboxyl group, an alkyl ester group having at least one hydroxyl group, an ether group, an amide group, a urethane group, a hydroxyl group or a C1 to C6 alkyl group having any of these groups).

2. A use according to claim 1, wherein the total amount of the monomers is 20 to 60 % by mass.

3. A use according to claim 1, wherein the total amount of the emulsifiers is 0.1 to 20 % by mass based on the total amount of the monomers.

4. A use according to claim 1, wherein the water-based polymer emulsion is one resulting from emulsion polymerization by one or more reactive polymerizing agents.

5. A cosmetic formulation containing a water-based polymer emulsion according to claim 1.

6. A use according to one of claims 1 to 4 wherein the cosmetic formulation is a hair treatment formulation.

7. A use according to one of claims 1 to 4 wherein the cosmetic formulation is a hair styling formulation.

8. A use according to one of claims 1 to 4 wherein the cosmetic formulation is a water-based nail polish formulation.

9. A use according to one of claims 1 to 4 wherein the cosmetic formulation is an eyeliner.

10. A use according to one of claims 1 to 4 wherein the cosmetic formulation is a mascara.

11. A use according to one of claims 1 to 4 wherein the cosmetic formulation is a sunscreen formulation.

12. A use according to claim 1, wherein the water-based polymer emulsion is a cationic water-based polymer emulsion, which is obtained by emulsion polymerization using one or more cationic emulsifiers.

13. A cosmetic formulation containing a cationic water-based polymer emulsion according to claim 12.

14. A use according to claim 12 wherein the cosmetic formulation is a hair styling formulation.

15. A use according to claim 12 wherein the cosmetic formulation is a hair treatment formulation.

16. A use according to claim 12 wherein the cosmetic formulation is a hair rinse formula.

## Patentansprüche

1. Verwendung einer wässrigen Polymeremulsion zur Herstellung einer kosmetischen Rezeptur, wobei die wäßrige Polymeremulsion durch Emulsionspolymerisation eines oder mehrerer kuppelnder Silanmonomere erhalten wird, die ausgewählt sind aus der aus den durch die Formel (1) dargestellten Monomeren (A) gebildeten Gruppe, eines oder mehrerer lipophiler radikalpolymerisierbarer Monomere, ausgewählt aus den durch die Formel (2) dargestellten Monomeren (B), und eines oder mehrerer hydrophiler radikalpolymerisierbarer Monomere, ausgewählt aus den durch die Formel (3) dargestellten Monomeren (C), wobei die Gesamtmenge der Monomeren (A) 0,001 bis 10 Masseprozent der gesamten Monomermenge beträgt, worin das Polymer mit den vom Monomer (A) abgeleiteten und darin verbliebenen reaktiven Silylgruppen in einem wässrigen Dispersionsmedium in einer solchen Konzentration dispergiert wird, die eine Vernetzung zwischen den Silylgruppen ermöglicht, oder in einer niedrigeren Konzentration; 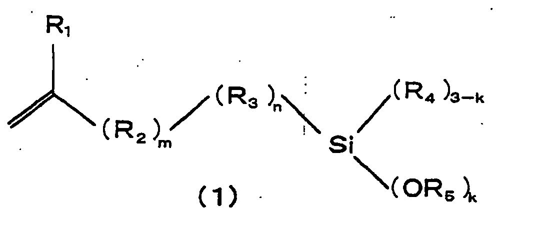
(worin R₁ ein Wasserstoffatom oder eine Alkylgruppe mit 1 bis 6 Kohlenstoffatomen ist, R₂ eine Gruppe, ausgewählt aus 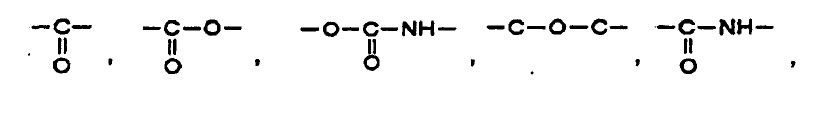
ist, R₃ eine Alkylengruppe mit 1 bis 6 Kohlenstoffatomen, R₄ und R₅ jeweils ein Wasserstoffatom oder eine Alkylgruppe mit 1 bis 6 Kohlenstoffatomen ist,
m und n jeweils 0 oder 1 und k eine ganze Zahl von 1 bis 3 ist); 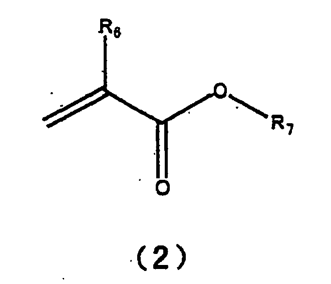
(worin R₆ ein Wasserstoffatom oder eine Methylgruppe ist, R₇ eine Alkylgruppe mit 1 bis 18 Kohlenstoffatomen, eine Alkylgruppe mit einem Fluoratom und 1 bis 18 Kohlenstoffatomen oder eine Alkylgruppe mit 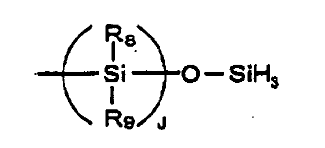
und 1 bis 18 Kohlenstoffatomen ist, wobei R₈ und R₉ jeweils ein Wasserstoffatom oder eine Methylgruppe und J eine ganze Zahl von 1 bis 18 ist); 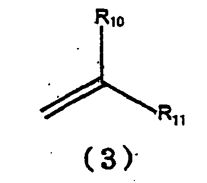
(worin R₁₀ ein Wasserstoffatom oder eine Methylgruppe ist, R₁₁ eine Carboxylgruppe, eine Alkylestergruppe mit mindestens einer Hydroxylgruppe, eine Ethergruppe, eine Amidgruppe, eine Urethangruppe, eine Hydroxylgruppe oder eine C₁-C₆-Alkylgruppe mit einer dieser Gruppen ist).

2. Verwendung nach Anspruch 1, wobei die Gesamtmenge der Monomere 20 bis 60 Masseprozent ist.

3. Verwendung nach Anspruch 1, wobei die Gesamtmenge der Emulgatoren 0,1 bis 20 Masseprozent der gesamten Monomermenge ist.

4. Verwendung nach Anspruch 1, wobei die wäßrige Polymeremulsion aus der Emulsionspolymerisation durch eines oder mehrere reaktive polymerisierende Mittel hervorgeht.

5. Kosmetische Rezeptur, enthaltend eine wäßrige Polymeremulsion nach Anspruch 1.

6. Verwendung nach einem der Ansprüche 1 bis 4, wobei die kosmetische Rezeptur eine Rezeptur zur Haarbehandlung ist.

7. Verwendung nach einem der Ansprüche 1 bis 4, wobei die kosmetische Rezeptur eine Rezeptur zum Haarstyling ist.

8. Verwendung nach einem der Ansprüche 1 bis 4, wobei die kosmetische Rezeptur eine wäßrige Nagellackrezeptur ist.

9. Verwendung nach einem der Ansprüche 1 bis 4, wobei die kosmetische Rezeptur ein Eyeliner ist.

10. Verwendung nach einem der Ansprüche 1 bis 4, wobei die kosmetische Rezeptur ein Mascara ist.

11. Verwendung nach einem der Ansprüche 1 bis 4, wobei die kosmetische Rezeptur eine Sonnenschutzrezeptur ist.

12. Verwendung nach Anspruch 1, wobei die wäßrige Polymeremulsion eine kationische wäßrige Polymeremulsion ist, die durch Emulsionspolymerisation mit einem oder mehreren kationischen Emulgatoren erhalten wird.

13. Kosmetische Rezeptur mit einer kationischen wäßrigen Polymeremulsion nach Anspruch 12.

14. Verwendung nach Anspruch 12, wobei die kosmetische Rezeptur eine Rezeptur zum Haarstyling ist.

15. Verwendung nach Anspruch 1, wobei die kosmetische Rezeptur eine Rezeptur zur Haarbehandlung ist.

16. Verwendung nach Anspruch 1, wobei die kosmetische Rezeptur eine Rezeptur zur Haarspülung ist.

## Revendications

1. Utilisation d'une émulsion polymère aqueuse dans la préparation d'une formulation cosmétique, dans laquelle on obtient l'émulsion polymère aqueuse par polymérisation en émulsion d'un ou de plusieurs monomères de couplage de type silane choisis dans le groupe constitué par les monomères (A) représentés par la formule (1), un ou plusieurs monomères lipophiles polymérisables par voie radicalaire choisis parmi les monomères (B) représentés par la formule (2) et un ou plusieurs monomères hydrophiles polymérisables par voie radicalaire choisis parmi les monomères (C) représentés par la formule (3), dans laquelle la teneur totale en monomères (A) est de 0,001 à 10 % en masse sur la base de la quantité totale des monomères, où le polymère ayant le groupe silyle réactif dérivé du monomère (A) restant à l'intérieur est dispersé à une certaine concentration ou moins, permettant à la réticulation entre lesdits groupes silyle de se produire dans un milieu de dispersion aqueux ; 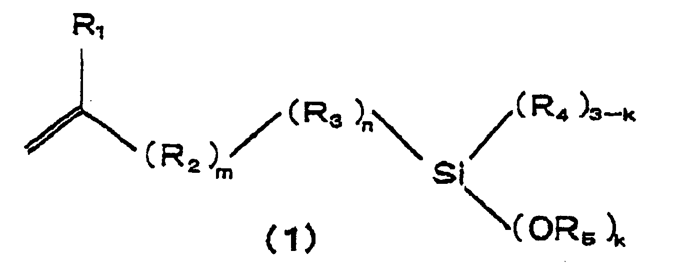
(dans laquelle R1 est un atome d'hydrogène ou un groupe alkyle ayant 1 à 6 atomes de carbone, R2 est un groupe choisi parmi 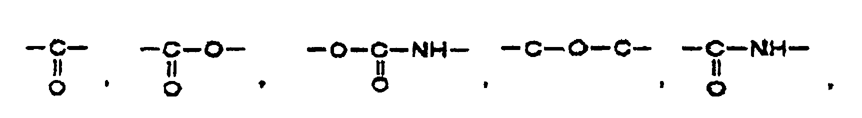
R3 est un groupe alkylène ayant 1 à 6 atomes de carbone, chacun des radicaux R4 et R5 est un atome d'hydrogène ou un groupe alkyle ayant 1 à 6 atomes de carbone,
chacun parmi m et n vaut 0 ou 1 et k est un entier valant 1 à 3) ; 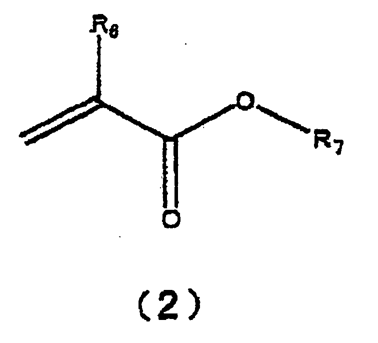
(dans laquelle R6 est un atome d'hydrogène ou un groupe méthyle, R7 est un groupe alkyle ayant 1 à 18 atomes de carbone, un groupe alkyle ayant un atome de fluor et 1 à 18 atomes de carbone ou un groupe alkyle ayant 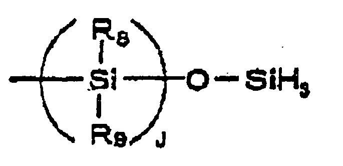
et 1 à 18 atomes de carbone, dans laquelle chacun des radicaux R8 et R9 est un atome d'hydrogène ou un groupe méthyle et J est un entier valant 1 à 18) ; 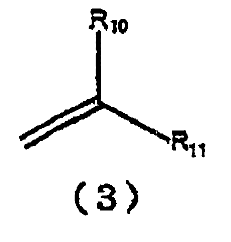
(dans laquelle R10 est un atome d'hydrogène ou un groupe méthyle, R11 est un groupe carboxyle, un groupe ester alkylique ayant au moins un groupe hydroxyle, un groupe éther, un groupe amide, un groupe uréthane, un groupe hydroxyle ou un groupe alkyle en C₁-C₆ ayant l'un quelconque de ces groupes).

2. Utilisation selon la revendication 1, dans laquelle la quantité totale des monomères est de 20 à 60 % en masse.

3. Utilisation selon la revendication 1, dans laquelle la quantité totale des émulsifiants est de 0,1 à 20 % en masse sur la base de la quantité totale des monomères.

4. Utilisation selon la revendication 1, dans laquelle l'émulsion polymère aqueuse est une émulsion résultant d'une polymérisation en émulsion avec un ou plusieurs agents de polymérisation réactifs.

5. Formulation cosmétique contenant une émulsion polymère aqueuse selon la revendication 1.

6. Utilisation selon l'une des revendications 1 à 4, dans laquelle la formulation cosmétique est une formulation capillaire traitante.

7. Utilisation selon l'une des revendications 1 à 4, dans laquelle la formulation cosmétique est une formulation pour le coiffage.

8. Utilisation selon l'une des revendications 1 à 4, dans laquelle la formulation cosmétique est une formulation aqueuse de vernis à ongles.

9. Utilisation selon l'une des revendications 1 à 4, dans laquelle la formulation cosmétique est un eyeliner.

10. Utilisation selon l'une des revendications 1 à 4, dans laquelle la formulation cosmétique est un mascara.

11. Utilisation selon l'une des revendications 1 à 4, dans laquelle la formulation cosmétique est une formulation d'écran solaire.

12. Utilisation selon la revendication 1, dans laquelle l'émulsion polymère aqueuse est une émulsion polymère aqueuse cationique obtenue par polymérisation en émulsion en utilisant un ou plusieurs émulsifiants cationiques.

13. Formulation cosmétique contenant une émulsion polymère aqueuse cationique selon la revendication 12.

14. Utilisation selon la revendication 12, dans laquelle la formulation cosmétique est une formulation pour le coiffage.

15. Utilisation selon la revendication 12, dans laquelle la formulation cosmétique est une formulation capillaire traitante.

16. Utilisation selon la revendication 12, dans laquelle la formulation cosmétique est une formule capillaire à rincer.
